# EUROPÄISCHE PATENTANMELDUNG

(11) **EP 1 010 393 A1**
(43) Veröffentlichungstag der Anmeldung: **21.06.2000**
(21) Anmeldenummer: 99124178.7
(22) Anmeldetag: 03.12.1999
(51) Int. Cl.: A61B 5/107, A43D 1/02

(54) **Vorrichtung zur dreidimensionalen Vermessung menschlicher Füsse**

(30) Priorität: 15.12.1998 DE 29822320 U
(71) Anmelder: FAGUS-GRECON GRETEN GMBH & CO. KG, 31061 Alfeld (Leine) (DE)
(72) Erfinder: Greten, Ernst, 31073 Delligsen (DE)
(74) Vertreter: Kosel, Peter, Dipl.-Ing.

(57) **Zusammenfassung**

Die Vorrichtung (1) weist einen Fußscanner (4) mit mehreren auf die Füße (2,3) gerichteten Strahlungsquellen (5) und Strahlungsempfängern (6) auf. Messsignale der Strahlungsempfänger (6) werden in eine Auswerteeinheit (8) eingegeben. Die Auswerteeinheit (8) wertet die Messsignale aus und verarbeitet sie. Entsprechende Daten in digitalisierter Form, gegebenenfalls zusammen mit anderen personenbezogenen Daten, werden in einem Speicherbereich (12;13) eines kartenförmigen Datenträgers (14) gespeichert.

## Beschreibung

Die Erfindung betrifft eine Vorrichtung gemäß dem Oberbegriff des Anspruchs 1.

Bei einer bekannten Vorrichtung dieser Art (Footscanner SC150 der Firma Shafir Production Systems Ltd., P.O.BOX 192, RA'ANANA 43100, Israel) können Ausgangssignale der Auswerteeinheit entweder auf einem Monitor sichtbar oder ausgedruckt werden. Es hat sich gezeigt, daß der Kunde solche Ausdrucke nicht ständig bei sich führt und insbesondere dann meist nicht zur Verfügung hat, wenn er neue Schuhe kaufen will.

In der US 4 916 296 A sind in den Fig. 1 bis 8 chronologisch mehrere an sich bekannte kartenförmige Datenträger dargestellt. Diese und auch die im Rest dieser Druckschrift verwendeten kartenförmigen Datenträger dienen nicht zur Speicherung und Auswertung der Daten dreidimensional vermessener menschlicher Füße.

Aus der DE 43 23 337 C2 ist es an sich bekannt, in ein Brillengestell einen miniaturisierten Festspeicher, wie ein EPROM oder ein EEPROM, als Datenträger für die Kennzeichnung des Brillengestells und für die persönlichen optischen sowie Dezentrationswerte der für das Brillengestell bestimmten Brillengläser einzulassen.

Der Erfindung liegt die Aufgabe zugrunde, dem Kunden einen Datenträger an die Hand zu geben, den er ohne besondere Umstände ständig bei sich führen kann und insbesondere dann zur Verfügung hat, wenn er neue Schuhe kaufen will.

Diese Aufgabe ist durch die Merkmale des Anspruchs 1 gelöst. Als Strahlungsquellen kommen insbesondere körperschonende Infrarotstrahler in Betracht. Den kartenförmigen Datenträger kann der Kunde wie seine übrigen Karten, z.B. Kredit-, Scheck- und Telefonkarten, ständig sicher bei sich führen. Die Daten auf dem Datenträger sind an jeder Stelle, die sich mit Verkauf und der Herstellung von Schuhen befaßt, leicht auf einem Monitor darstellbar oder auszudrucken. Der Fachhändler oder der Schuhhersteller können sich auf diese Weise sofort ein Bild über die Maßdaten der Füße des Kunden machen und um so schneller und sicherer bei der Auswahl oder Herstellung neuer Schuhe beraten. So kann der Schuhfachhändler genau sehen, ob angesichts der sichtbar gemachten Fußmeßdaten des Kunden auf Lager befindliches Schuhwerk in Betracht kommt, oder ob angesichts ausgefallener Fußmeßdaten eine Sonderanfertigung von Schuhen richtiger ist. Im Extremfall kann er die Anfertigung orthopädischen Schuhwerks empfehlen. Der Fachhändler kann z.B. auch sehen, daß ein Fuß des Kunden größer ist als der andere. Bei einem zur Auswahl in Betracht kommenden Schuhpaar kann er dann zunächst den Schuh an dem größeren Fuß anprobieren. Wenn dieser paßt, ist davon auszugehen, daß auch der andere Schuh an den kleineren Fuß paßt oder passend gemacht werden kann. Diese Vorsichtung kann insbesondere hinsichtlich der Schuhgröße und der Schuhweite geschehen. In allen Fällen läßt sich die Auswahl am Lager befindlicher Schuhe erheblich abkürzen und sicherer gestalten und auch sofort sagen, ob die speziellen Fußmeßdaten des Kunden außerhalb der Lagerware liegen und deshalb Spezialanfertigung oder gar orthopädische Anfertigung verlangen. So kommen der Schuhfachhandel und der Kunde insgesamt sicherer und schneller zum Ziel.

Der Datenträger gemäß Anspruch 2 läßt sich besonders einfach im Rahmen sonstiger Karten des Kunden mitführen.

Die Ausbildungen des Speicherbereichs gemäß Anspruch 3 oder 4 wählt man je nach dem Umfang der zu speichernden Datenmenge und nach der Art der Speicherung und Handhabung der Daten.

Die Daten gemäß Anspruch 5 sind für alle Beteiligten von besonderer Bedeutung.

Die Merkmale des Anspruchs 6 erleichtern oder ermöglichen die Nutzung der gespeicherten Daten in vielen Ländern der Welt.

Die Merkmale des Anspruchs 7 helfen dem Schuhfachhändler insbesondere bei unterschiedlichen Füßen eines Kunden die Auswahl abzukürzen.

Gemäß Anspruch 8 wird der Schuhfachhändler oder Schuhhersteller in die Lage versetzt, die Fußmeßdaten des Kunden schnell und sicher zur Kenntnis zu nehmen.

Diese und weitere Merkmale der Erfindung werden nachfolgend anhand der in den Zeichnungen dargestellten Ausführungsbeispiele näher erläutert. Es zeigt:
Fig. 1 Die schematische Darstellung einer Vorrichtung und
Fig. 2 die schematische Darstellung eines weiteren Teils einer Vorrichtung.

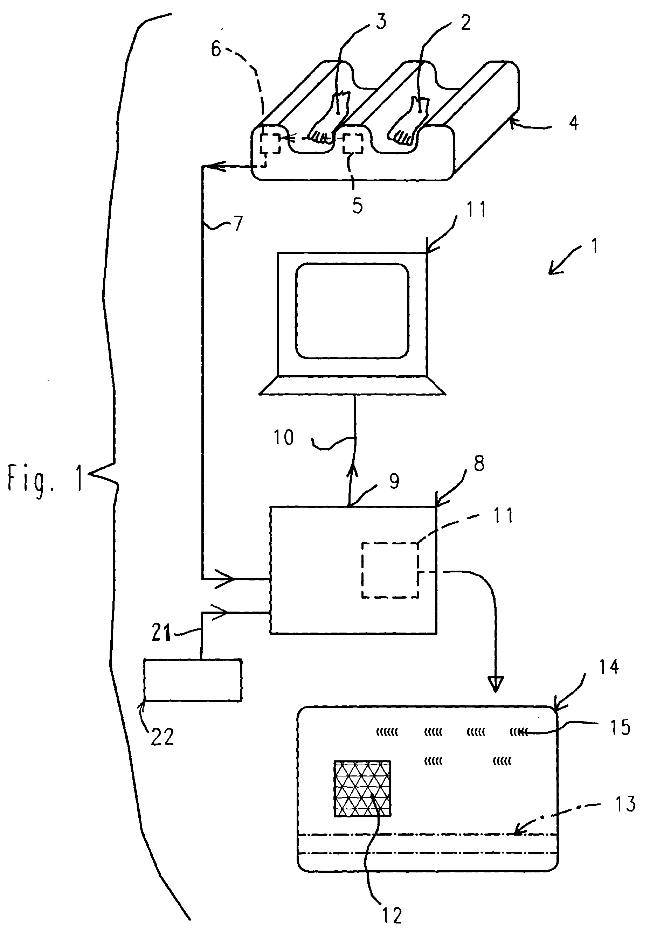

Fig. 1 zeigt eine Vorrichtung 1 zur dreidimensionalen Vermessung menschlicher Füße 2 und 3. Die Vorrichtung 1 weist einen Fußscanner 4 mit mehreren Strahlungsquellen 5 und mehreren Strahlungsempfängern 6 auf, von denen Fig. 1 jeweils nur eine/einer gestrichelt angedeutet sind. Jede Strahlungsquelle 5 sendet Strahlung, z.B. Infrarotstrahlung, in Richtung eines der Füße 2, 3 und des zugehörigen Strahlungsempfängers 6. In Abhängigkeit von der Form des Fußes 2, 3 bildet der Strahlungsempfänger Meßsignale, die über eine Leitung 7 in eine Auswerteeinheit 8 eingegeben werden. In der Auswerteeinheit 8 werden die Meßsignale digitalisiert und verarbeitet. Ein Ausgang 9 der Auswerteeinheit 8 ist über eine Leitung 10 mit einem Monitor 11 verbunden, um Meßwerte und/oder daraus verarbeitete Informationen sichtbar zu machen. Über eine Leitung 21 können mit einer Eingabeeinheit 22 weitere personenbezogene Daten, wie z.B. Hinweise auf spezielle Leisten, besondere Fußmaße oder die gesamten Meßdaten des Fußscanners 4 und Hinweise für den Fachhändler über das Kaufverhalten des Kunden, z.B. welches Modell er gekauft hat, 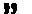 kritischer" Kunde usw., in die Auswerteeinheit 8 eingegeben werden.

In der Auswerteeinheit 8 oder durch die Auswerteeinheit 8 gesteuert ist eine Kodiereinheit 11 vorgesehen. Durch die Kodiereinheit 11 werden aus den Meßsignalen gewonnene Daten und ggf. weitere personenbezogene Daten in einem Speicherbereich 12 und/oder 13 in digitalisierter und ggf. kodierter Form gespeichert Bei dem Speicherbereich 12 kann es sich z.B. um ein EPROM oder EEPROM handeln. Auf dem kartenförmigen Datenträger 14 können außerdem mit dem Auge lesbare Bereiche 15 vorgesehen sein, die z.B. den Namen des Kunden, die Schuhgröße in den gängigsten internationalen Größensystemen, die Schuhweite und/oder Empfehlungen zur Anprobe, wie 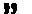 Vorzugsweise linken Schuh probieren", 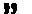 Achten Sie auf Zehenhöhe" oder 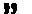 Achten Sie auf genug Zehenbreite", enthalten können.

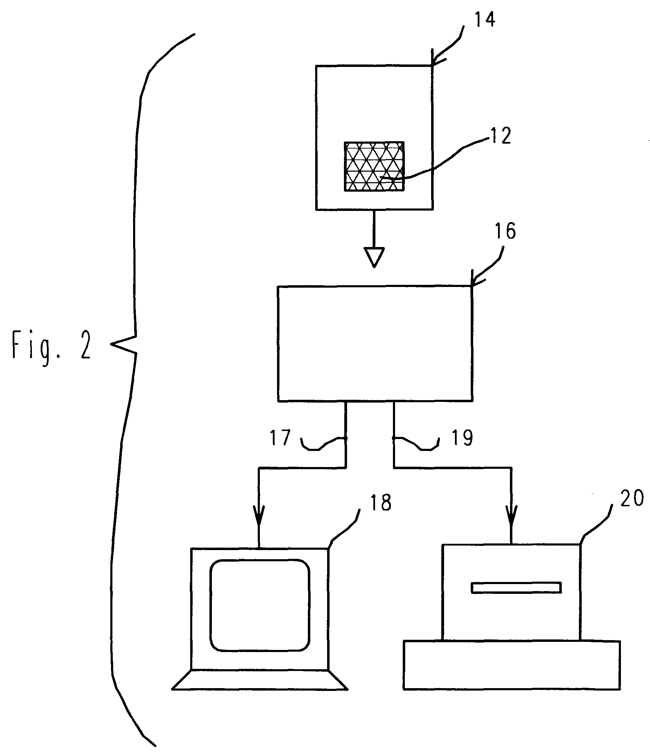

Einen solchermaßen mit den Meßdaten seiner Füße und daraus gewonnenen sekundären Daten, wie z.B. Schuhgröße und Schuhweite, und ggf. mit weiteren personenbezogenen Daten geladenen Datenträger kann der Kunde stets bei sich führen. Besucht der Kunde z.B. ein Schuhfachgeschäft, wird der Datenträger 14 gemäß Fig. 2 in ein in dem Geschäft stationiertes Lesegerät 16 eingegeben. Die durch das Lesegerät 16 auf diese Weise gelesenen Daten des Datenträgers 14 gelangen über eine Leitung 17 in einen Monitor 18 und/oder über eine Leitung 19 in einen Drucker 20, der die Daten dann auf einen Ausdruck liefert. Der Fachhändler kann den Datenträger 14 erstellen oder die Eintragungen und Speicherungen auf dem Datenträger 14 ändern.

## Patentansprüche

1. Vorrichtung zur dreidimensionalen Vermessung menschlicher Füße (2,3),
mit einem Fußscanner (4), der mehrere auf die Füße (2, 3) gerichtete Strahlungsquellen (5) und Strahlungsempfänger (6) aufweist,
und mit einer Auswerteeinheit (8), in die Meßsignale der Strahlungsempfänger (6) eingebbar sind,
dadurch gekennzeichnet, daß die Meßsignale durch die Auswerteeinheit (8) auswertbar und verarbeitbar und entsprechende Daten in digitalisierter Form, gegebenenfalls zusammen mit anderen personenbezogenen Daten (vgl. 20), in einem Speicherbereich (12;13) eines kartenförmigen Datenträgers (14) speicherbar sind.

2. Vorrichtung nach Anspruch 1,
dadurch gekennzeichnet, daß der Datenträger (14) nach Art einer Kreditkarte ausgebildet ist.

3. Vorrichtung nach Anspruch 1 oder 2,
dadurch gekennzeichnet, daß der Speicherbereich (13) einen Magnetstreifen aufweist.

4. Vorrichtung nach einem der Ansprüche 1 oder 2,
dadurch gekennzeichnet, daß der Speicherbereich (12) einen miniaturisierten Festspeicher, wie z.B. ein EPROM oder ein EEPROM, aufweist.

5. Vorrichtung nach einem der Ansprüche 1 bis 4,
dadurch gekennzeichnet, daß es sich bei den gespeicherten Daten z.B. um die Schuhgröße, die Fußlänge, die Fußweite, den Ballenumfang, eine kurze Ferse und Spanndaten handelt.

6. Vorrichtung nach einem der Ansprüche 1 bis 5,
dadurch gekennzeichnet, daß die Daten jeweils in den unterschiedlichen, international gebräuchlichen Maß- und Bezeichnungssystemen gespeichert sind, z.B. Schuhgrößen im Pariser Stich (z.B. 44), in englischen Größen (z.B. 9 1/2) und im Mondo-Point (d.h. Fußlänge in mm, z.B. 480 mm), und z.B. Fußweiten in GH und im U.S.-Bezeichnungssystem (z.B. Weite AA).

7. Vorrichtung nach einem der Ansprüche 1 bis 6,
dadurch gekennzeichnet, daß die Daten für jeden Fuß (2,3) gesondert gespeichert sind.

8. Vorrichtung nach einem der Ansprüche 1 bis 7,
dadurch gekennzeichnet, daß der kartenförmige Datenträger (14) in ein Lesegerät (16) eingebbar ist,
und daß durch das Lesegerät (16) gelesene, auf dem Datenträger (14) gespeicherte Daten auf einem Monitor (18) darstellbar und/oder durch einen Drucker (20) ausdruckbar sind.
